# EUROPEAN PATENT APPLICATION

(11) **EP 2 138 201 A2**
(43) Date of publication of application: **30.12.2009**
(21) Application number: 09003327.5
(22) Date of filing: 06.03.2009
(51) Int. Cl.: A61M 25/00, B29C 47/20

(54) **Extrusion molding die and extrusion molding method for molding a medical treatment tube**

(30) Priority: 25.06.2008 JP 2008165730
(71) Applicant: Covidien AG, 8212 Neuhausen am Rheinfall (CH)
(72) Inventor: Fujiwara, Norifumi, Tokyo 158-8615 (JP); Ohigawa, Atsushi, Tokyo 158-8615 (JP)
(74) Representative: Olsson, Carl H.S.

(57) **Abstract**

An extrusion molding die (20), for extruding a medical treatment tube (10), in which is formed a flow path (13a), and the like, between dividing wall portions (12), is structured from an outer shape molding die, wherein indented portions (25b,27a, and 28a) are formed, and a pin (22) that is provided with a land portion (23). Additionally, a space that is formed between the outer shape molding die and the pin (22) is structured from a rear portion side molding space portion, which forms the molding material into a cylinder that is pointed at the tip, and a tip side molding space portion, provided with a cross-sectional shape capable of forming the medical treatment tube (10). In addition, in the cross-sectional shape of the tip side molding space portion, the cross-sectional shape at the tip portion is the same as the cross-sectional shape of the medical treatment tube (10), and the cross-sectional shape of the rear side portion is shaped as the portion that shapes the flow path (13a, etc.,) in the land portion (23), with the portions that shape the flow paths (13a and 13c) removed.

## Description

### (Technical Field)

The present invention relates to an extrusion molding die and extrusion molding method for molding a medical treatment tube used in order to drain fluid from a patient wound or to provide a medicinal solution to a patient wound.

### (Prior Art)

Conventionally, medical treatment tubes have been used for draining drainage that has accumulated in patient wounds. Among this type of medical treatment tubes there are those that are structured as a silicone molded body, wherein the base end side portion is formed into a cylindrical shape, and the front end side portion, which is resident in the patient wound portion, is structured from a plurality of partitioned flow paths around a central axis. Additionally, in the front end portion of this medical treatment tube, slits are formed by cutting away, in the axial direction, the outer peripheral side portions of the wall portions that form the individual flow paths. The provision of these slits increases the size of the opening portions of the medical treatment tube, enabling the efficient aspiration of the drainage fluids. (See, for example, Patent Reference 1.)

The cross-sectional shape of the front end side portion of this type of medical treatment tube (wound drain catheter) is a shape wherein four flow paths are formed by connecting, to the inner surface of the portion that is formed into a ring-shaped, the respective front end portions of the pieces of a portion that is formed into a cross shape, where the portions of the ring-shaped portion corresponding to the intervals between each of the pieces are cut away to form slits, to connect each of the four flow paths to the outside. There is also a shape wherein the three flow paths are formed connected respectively to each of the front end portions of pieces that extend in three directions from the center of the ring shape to the inner surface of the ring-shaped portion, where the portions of the ring-shaped portion corresponding to the intervals between each of the pieces are cut away to form slits, to connect each of the three flow paths to the outside.

Additionally, as a method for manufacturing a conventional medical treatment tube as described above, there is, for example, forming through first extrusion molding a tube wherein the cross-sectional shape forms four flow paths through connecting, to the inner surface of a portion that is formed into a ring shape, each of the front end portions of the pieces of a cross shape. There is a method where, following this, the medical treatment tube is formed through providing slits by cutting away, and a processing treatment, the portions of the ring-shaped portion that corresponds to the intervals between the pieces. Furthermore, there is also a method of forming the medical treatment tube that is provided with slits directly through the extrusion molding.
(Patent Reference 1) Japanese Unexamined Patent Application Publication H2-17185

### (Disclosure of the Invention)

However, in these conventional methods for manufacturing medical treatment tubes, the case wherein the slits are formed through a processing treatment has a problem in that this increases the number of manufacturing processes and increases the cost. Furthermore, in the case of forming the medical treatment tube in the final form all at once in the extrusion molding, if a soft resin is used as the molding material, then typically the material does not slide easily on that the metal sleeve of the extrusion molding die, making it difficult to perform the molding using, for example, the vacuum sizing method. Furthermore, even with soft resins, the viscosity is high when a silicone resin (a non-thermoplastic resin) is used, making it easy to mold into a shape that is close to the shape of the molding space in the extrusion molding die, but when a molding material made from a soft thermal plastic resin, such as soft polyurethane or soft polyvinyl chloride, is used, it is difficult to perform molding, using the conventional extrusion molding method, in the same way as when the silicone resin is used.

The present invention is the result of contemplation on the circumstances described above, and the object thereof is to provide an extrusion molding die and an extrusion molding method for molding a medical treatment tube, capable of molding a medical treatment tube with complex shapes with excellent precision, using a molding material made out of resin.

In order to achieve the aforementioned object, the structural characteristics of the extrusion molding die for molding the medical treatment tube, as set forth in the present invention, are in the provision of
an extrusion molding guide for molding a medical treatment tube wherein a plurality of flow paths are formed around an axial portion, comprising:
an outer shape molding die wherein a hollow portion is formed passing through from the rear portion side to the front end side; and
an inner shape molding pin, disposed within the hollow portion of the outer shape molding die, is structured with a land portion so as to form a front end side molding space that is provided with a cross-sectional shape for forming a rear portion side molding space portion between the inner shape molding pin and the inner peripheral wall of the side portion of the rear portion of the hollow portion to form the molding material into a cylinder that is pointed at the front end, wherein the front end portion forms the medical treatment tube between the inner shape molding pin and the inner peripheral surface of the front end side portion of the hollow portion; wherein:
in the cross-sectional shape of the front end side molding space portion, the cross-sectional shape of the front end portion is identical to the cross-sectional shape of the medical treatment tube, and, with the exception of at least one portion of each portion that corresponds to the plurality of flow passages in the land portion, the cross-sectional shape of the rear portion side portion is empty space wherein the molding material can pass therethrough.

In the extrusion molding die for molding the medical treatment tube as set forth in the present invention, structured as described above, the portion side portion of the space for molding, formed by the outer shape molding die and the inner shape molding pin, is formed into a rear portion side molding space portion that is cylindrical and pointed at the front end, where the molding material that is molded gradually approaches the thickness of the medical treatment tube from the cylindrical shape that has a large diameter. Additionally, among the molding space portions, the cross-sectional shape of the front end side molding space portion that is positioned on the front end side has a shape that is different from those of the rear side portion and the front end portion. That is, the cross-sectional shape of the rear portion side portion of the front end side molding space portion is a shape wherein the portion that forms at least one of the flow paths of the portion that forms the plurality of flow paths has been removed in the land portion, where the cross-sectional shape of the front end portion is the same as the cross-sectional shape of the medical treatment tube.

In this way, the cross-sectional shape of the rear portion side portion of the front end side molding space portion is a shape wherein the portion that forms at least one of the flow paths of the portion that forms the plurality of flow paths has been removed in the land portion, and thus that portion becomes a space through which the molding material can pass together with the other portions of the space. Because of this, the rear portion side portion has a greater volume of molding material per cross-sectional area than the front end portion of the front end side molding space portion, enabling the provision of adequate molding material to the front end portion from the rear portion side portion of the front end side molding space portion. As a result, the situation wherein the volume of molding material that is provided to the front end portion of the front end side molding space portion is reduced, preventing the molding of a medical treatment tube with adequate wall thickness, does not occur. The result is that the molded body that is extruded to the outside from the front end side molding space portion will be an excellent medical treatment tube, provided with the cross-sectional shape of the design.

Additionally, the extrusion molding device to which this type of extrusion molding die is attached is provided with a heating cylinder for heating the molding material which is fed into the extrusion molding die, a screw for pushing, to the extrusion molding die side, the molding material that has been placed into the heating cylinder. Furthermore, the provision of the portion with a large cross-sectional shape in the front end side molding space portion enables the rotational torque of the screw to be reduced, enabling the temperature of the molding material to be reduced correspondingly. The result is that is possible to increase the viscosity of the molding material, making it possible to stabilize the shape of the medical treatment through that is molded.

Another structural characteristic of an extrusion molding die for molding a medical treatment tube as set forth in the present invention is that the front end portion of the outer shape molding die is structured from a plate portion provided with an external surface forming hollow portion for forming a surface along the outside of the molding material that moves from the rear portion side molding space portion, wherein a portion of the land extends to a predetermined position within the external surface forming hollow portion of the plate portion so as to form a flow path, the peripheral surfaces thereof being enclosed by the medical treatment tube that is formed, at the position of the land portion that is positioned within the plate portion.

In the extrusion molding die for molding the medical treatment tube, the front end portion of the outer shape molding die is structured from a plate that is provided with an external surface molding hollow portion for forming a surface along the outside of the medical treatment tube, where a portion of the land portion extends into the external surface molding hollow portion of the plate portion. Because of this, it is possible to shape a predetermined flow path, of the plurality of flow paths that are provided in the medical treatment tube, into a shape wherein the peripheral surfaces, aside from both ends surfaces, are closed. In this case, there may be a plurality of portions of the land portion that extends into the external surface molding hollow portion of the plate portion. In accordance therewith, it is possible to form a plurality of flow paths, with the peripheral surfaces thereof being closed, in the medical treatment tube.

Note that when a site is provided in a predetermined portion of the medical treatment tube and the flow path connects to the outside through the slit, a blocking portion is provided in a specified portion of the space on the outer peripheral side of the portion corresponding to the flow path in the hollow portion for forming the outer surface of the plate portion. As a result, it is possible to form a slit in the portion corresponding to the blocking portion in the medical treatment tube. Consequently, if slits are provided corresponding to all of the flow paths in the medical treatment tube, it is possible to form the flow paths, provided with slits, in only the hollow portion for forming the outer surface of the plate portion, without extending to be front end portion of the land portion within the hollow portion for forming in the outer surface of the plate portion.

At the front end of this outer shape molding die, the portion that connects to the outer portion through the slit is formed through the outer shape molding die, even if the surface is one wherein a flow path is formed. Moreover, a predetermined portion of the outer peripheral portion of the land portion is caused to protrude circumferentially to contact a specific portion of a hollow portion for forming the outer surface of the plate portion or a hollow portion of the outer shape molding die, or a small gap into which the molding material is not able to penetrate is formed with the inner peripheral surface of the hollow portion or the inner peripheral surface of the hollow portion for forming the outer shape, to thereby be able to form a slit in the portion corresponding to the portion wherein the land portion protrudes in the medical treatment tube.

Another structural characteristic of an extrusion molding die for molding a medical treatment tube as set forth in the present invention is that the cross-sectional shape of the front end portion of the front end side molding space portion can form four flow paths by connecting each of the front end portions of space portions of a cross shape to the inner surface of the space portion of a ring shape, formed so that predetermined portions of the space portion of the ring shape may be blocked so that at least one of the four flow paths that can be formed connects to the outside. This makes it unnecessary to provide a slit through mechanical processing after molding, because it is possible to mold, through a single extrusion molding step, a medical treatment tube having an irregular shape wherein a slit is provided to connect a predetermined flow path to the outside. The result is that it is possible to reduce the number of manufacturing processes and to reduce the manufacturing cost. Furthermore, the ring-shaped space portion in this case is closed, and either a slit may be provided for a single flow path, were slits may be provided for a plurality of two through four flow paths. Moreover, the ring-shaped in this case may be a shape such as a circular shape or an elliptical shape, etc.

Another structural characteristic of an extrusion molding die for molding a medical treatment tube as set forth in the present invention is that the cross-sectional shape of the front end portion of the front end side molding space portion can form four flow paths by connecting each of the front end portions of space portions of a "H" shape to the inner surface of the space portion of a ring shape, formed so that predetermined portions of the space portion of the ring shape may be blocked so that at least one of the four flow paths that can be formed connects to the outside. This makes it unnecessary to provide a slit through mechanical processing after molding, because it is possible to mold, through a single extrusion molding step, a flat medical treatment tube wherein a slit is provided to connect a predetermined flow path to the outside. The result is that it is possible to reduce the number of manufacturing processes and to reduce the manufacturing cost. Furthermore, the ring-shaped space portion in this case is closed, and either a slit may be provided for a single flow path, were slits may be provided for a plurality of two through four flow paths. Moreover, the ring-shaped in this case may be a shape such as a circular shape or an elliptical shape, etc.

Additionally, another structural characteristic of an extrusion molding die for molding a medical treatment tube as set forth in the present invention is that the molding material is a soft thermoplastic resin. This makes it possible to obtain an excellent medical treatment tube using a soft thermoplastic resin wherein, normally, it has been difficult to form a medical treatment tube having a complex shape.

A structural characteristic of an extrusion molding method for a medical treatment tube as set forth in the present invention is a method of extrusion molding a medical treatment tube for molding the medical treatment tube using the aforementioned extrusion molding die comprising: a pointed cylinder molding process for forming the molding material into a cylinder that is pointed at the front end through disposing the inner shape molding pin in the hollow portion of the outer shape molding die and passing the molding material through a rear portion side molding space portion that is a cylinder that is pointed at the front end; an intermediate shape molding process for molding, at the rear portion side portion of the front end side molding space portion, the cylindrical cross-sectional shape of the molding material, which has been formed in the pointed cylinder molding process, into an intermediate shape between the cross-sectional shape of the medical treatment tube and the cylindrical cross-sectional shape, which is a shape wherein the molding material is filled into a portion wherein at least one of the plurality of flow paths is formed; and a final shape molding process for molding, at the front end portion of the front end side forming space portion, the cross-sectional shape of the molding material that has been formed in the intermediate shape molding process into the cross-sectional shape of the medical treatment tube.

The extrusion molding method for a medical treatment tube having this structure has a pointed cylinder molding process for forming the molding material into a cylinder that is pointed at the front end, an intermediate shape molding process for molding the cross-sectional shape of the material for molding, which has a pointed cylindrical shape, into an intermediate shape between the circular cross-sectional shape and the cross-sectional shape of the medical treatment tube, and a final shape molding process for molding the cross-sectional shape of the molding material that has been molded in the intermediate shape molding process into the cross-sectional shape of the medical treatment tube. Because of this, the cross-sectional shape of the molding material is first transformed from the circular cross-sectional shape of the molding space portion on the rear portion side, which is formed between the hollow portion of the outer shape molding die and the inner shape molding pin, into an intermediate shape that is near to the cross-sectional shape of the medical treatment tube, after which it is transformed into the cross-sectional shape of the final medical treatment tube from this intermediate shape.

Through this, it is possible to reduce the change there will be to the shape through a single transformation of the molding material, making it possible to mold, with high precision, a medical treatment tube with a complex shape, even when the molding material does not slide easily on the mold. Furthermore, because the intermediate shape is formed with the exclusion of at least one portion of the portions that correspond to the plurality of flow paths in the land portion, there is a large volume of the molding material that passes through the intermediate portion, supplying an adequate amount of molding material to the final shape molding process. As a result, the situation wherein the volume of molding material that is provided to final molding process is reduced, preventing the molding of a medical treatment tube with adequate wall thickness, does not occur.

Another structural characteristic of an extrusion molding method for a medical treatment tube as set forth in the present invention is a method of extrusion molding a medical treatment tube as set forth in claim 6, wherein the cross-sectional shape of the molding material that is formed in the pointed cylinder molding process is formed into a shape provided with four flow paths, each connected to the respective front end portion of the axial portions that are formed in a cross shape on the inner surface of a circular ring-shaped portion, wherein a predetermined portion of the circular ring-shaped portion is cutaway so that at least one of the four flow paths is connected to the outside. This makes it unnecessary to provide a slit through mechanical processing after molding, because it is possible to mold, through a single extrusion molding step, a tube having an irregular shape wherein a slit is provided. Moreover, the present invention enables a reduction in the number of manufacturing processes and a reduction in the manufacturing cost.

Yet another structural characteristic of an extrusion molding method for a medical treatment tube as set forth in the present invention is a method of extrusion molding a medical treatment tube as set forth in claim 6, wherein the cross-sectional shape of the molding material that is formed in the pointed cylinder molding process is formed into a shape provided with four flow paths, each connected to the respective front end portion of the axial portions that are formed in a "H" shape on the inner surface of a elliptical ring-shaped portion, wherein a predetermined portion of the elliptical ring-shaped portion is cutaway so that at least one of the four flow paths is connected to the outside. This makes it unnecessary to provide a slit through mechanical processing after molding, because it is possible to mold, through a single extrusion molding step, a flat tube wherein a slit is provided. The result is that it is possible to reduce the number of manufacturing processes and to reduce the manufacturing cost.

Additionally, yet another structural characteristic of an extrusion molding process for molding a medical treatment tube as set forth in the present invention is that the molding material is a soft thermoplastic resin. This makes it possible to obtain an excellent medical treatment tube using a soft thermoplastic resin wherein, normally, it has been difficult to form a medical treatment tube having a complex shape.

### (Most Preferred Means for Embodying the Invention)

### (First Example of Embodiment)

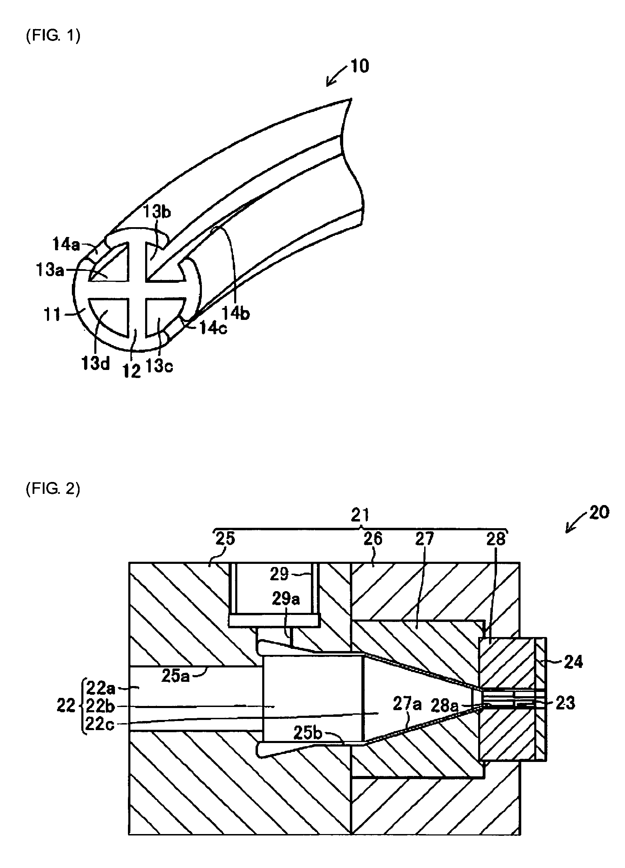

An extrusion molding die and an extrusion molding process for molding a medical treatment tube as set forth in the first example of embodiment according to the present invention will be explained below using the figures. FIG. 1 illustrates a medical treatment tube 10 formed through this example of embodiment. The medical treatment tube 10 is structured from a molded body made from polyurethane (a soft thermoplastic resin as set forth in the present invention), structured from an essentially tubular outer peripheral portion 11, dividing wall portion 12 (the axial portion in the present invention), having a cross-shaped cross-sectional shape, fabricated along the axial direction of the inner portion of the outer peripheral portion 11. The front end portion of each piece that forms the dividing wall portion 12 is each connected to the inner surface of the outer peripheral portion 11, dividing the inner portion of the outer peripheral portion 11 into four flow paths 13a, 13b, 13c, and 13d. Additionally, slits 14a, 14b, and 14c for connecting the respective flow paths 13a, 13b, and 13c, said slits corresponding to the flow paths 13a, 13b, and 13c in the outer peripheral portion 11, are each formed parallel to the central axis of the dividing wall portion 12.

A medical treatment tube 10 is structured in this way can be obtained through molding, using the extrusion molding die 20 illustrated in FIG. 2, a soft thermoplastic resin material made from polyurethane. This extrusion molding die 20 comprises a die main unit 21, a pin 22 that is disposed within the die main unit 21, a land portion 23 that is formed on the front end of the pin 22, and a plate portion 24 that is provided on the front end portion of the die main unit 21. The outer shape molding die in the present invention is structured from the die main unit 21 and the place portion 24, and the inner shape molding pin in the present invention is structured from the pin 22 that is provided with the land portion 23.

The die main unit 21 comprises a pin holder portion 25, made from an essentially rectangular block that is disposed on the rear portion side (the left side in FIG. 2, where, in the explanation below, the left side of FIG. 2 shall be the rear portion side, and the right side shall be the front portion side or the front end side), and a bushing holder portion 26 that is disposed on the front side of the pin holder portion 25, where bushings 27 and 28 are disposed on the inside of the bushing holder 26. A stepped, essentially round, cylindrical hole portion is formed on the inside of the pin holder portion 25 passing through from front to back, where the rear portion side of the hole portion structures a pin holding hole 25a, for holding the rear portion side portions of the pin 22, where the front portion side thereof forms a hollow portion 25b for accommodating the center portion of the pin 22.

The diameter of the hollow portion 25b is slightly larger than the diameter of the pin holding hole 25a, and a step is formed between the pin holding hole 25a and the hollow portion 25b. Moreover, the rear portion side portion of the hollow portion 25b is formed from an inclined peripheral surface that narrows at the front end from the back end towards the front end so that the front portion side portion of the hollow portion 25b is structured from a peripheral edge having an essentially constant diameter. Additionally, a material channel 29a, which extends from a material inlet 29 that is formed on the top surface of the pin holder portion 25, is connected to the upper portion of the rear end side portion of the hollow portion 25b. That is, the material channel 29a is connected perpendicularly relative to the hollow portion 25b.

A hollow portion to which a two-stepped bushing, wherein the front portion side is of a somewhat narrower diameter then the rear portion side, is attached on the inside of the bushing holder portion 26, passing through the center portion of the bushing holder portion 26 in the front-back direction. Furthermore, a square bushing 27 is disposed with the rear surface thereof in contact with the surface of the pin holder portion 25 on the rear portion side of the hollow portion for attaching the bushing, where a square bushing 28, which is smaller than the bushing 27, is disposed on the front side of the bushing 27 in the hollow portion for attaching the bushing in a state wherein the front front end portion of the square bushing 28 protrudes from the bushing holder 26. Furthermore, a hollow portion having a shallow depth (towards the rear) is formed in the center of the front and face of the bushing 27, where the rear portion of the bushing 28 is disposed within this hollow portion.

An essentially conical hollow portion 27a, with a smaller diameter towards the front portion side and a larger diameter towards the rear portion side, is formed from the center of the rear and face to the center of the front and face of the bushing 27, where, in the rear end portion of this hollow portion 27a is formed a slight portion having a constant diameter. The diameter of the rear end portion of the indented portion 27a is set so as to be identical to the diameter of the front end portion of the hollow portion 25b. A small diameter cylindrical hollow portion 28 a is formed from the center of the rear end face of the bushing 28 to the center of the front end face thereof, where the diameter of this hollow portion 28a is set so as to be identical to the diameter of the front end portion of the hollow portion 27a. Furthermore, the pin holding hole 25a, the hollow portion 25b, the hollow portion 27a, and the hollow portion 28a are all disposed so that the central axes thereof are all positioned collinearly.

The pin 22 is structured from: a cylindrical securing portion 22a that is secured within the pin holding hole 25a of the pin holder portion 25; a cylindrical straight portion 22b, which extends forward from the front end of the securing portion 22a, is short in the axial direction, and has a diameter that is slightly larger than that of the securing portion 22a; a cylindrical tapered portion 22c, which extends forward from the front end of the straight portion 22b and becomes gradually thinner towards the front end side; and the land portion 23. Furthermore, a gap through which the molding material passes is formed between the outer peripheral surface of the straight portion 22b and the inner peripheral surface of the hollow portion 25b, and between the outer peripheral surface of the tapered portion 22c and the inner peripheral surface of the hollow portion 27a, where the molding space portion on the rear portion side, as set forth in the present invention, is formed by this gap.

Furthermore, the land portion 23 that forms the front end portion of the pin 22 is disposed at the front end portion of the tapered portion 22c, as illustrated in FIG. 3 and FIG. 4, and is formed in two rod-shape units as illustrated in FIG. 5 through FIG. 7. That is, the rear end side portion of the land portion 23, as illustrated in FIG. 6, is structured from two rod shaped molded portions 23a and 23b that are wedge shaped in the cross-sectional shape thereof. These two rod shaped molded portions 23a and 23b are formed opposite each other, at a gap of 180° in the circumferential direction along the circular arc shaped portion of the outer periphery of the front end surface of the tapered portion 22c, and are disposed in parallel with the axial direction.

The portion other than the front end portion of the rod shaped portion 23a in this land portion 23 is disposed within the hollow portion 28a of the bushing 28, forming a large gap between the outer peripheral surface of the land portion 23 and the inner peripheral surface of the hollow portion 28 a (including between the rod shaped molded portions 23a and 23b). The rear portion side portion of the front end side molding space portion as set forth in the present invention is formed from this gap. That is, the portions that form the flow paths 13a and 13c, of all of those flow paths 13a, etc., that are provided in the medical treatment tube 10, are not provided on the rear end side portion of the front end side molding space portion. Consequently, when the molding material is supplied from the material inlet 29, this molding material is formed into a round cylinder when it enters into the hollow portion 25b after passing through the material channel 29a, and is formed into a pointed round cylinder in the hollow portion 27a. Furthermore, in the hollow portion 28a, the molding material fills the portion on the inside portion of the hollow portion 28a with the exception of the rod shaped portions 23a and 23b.

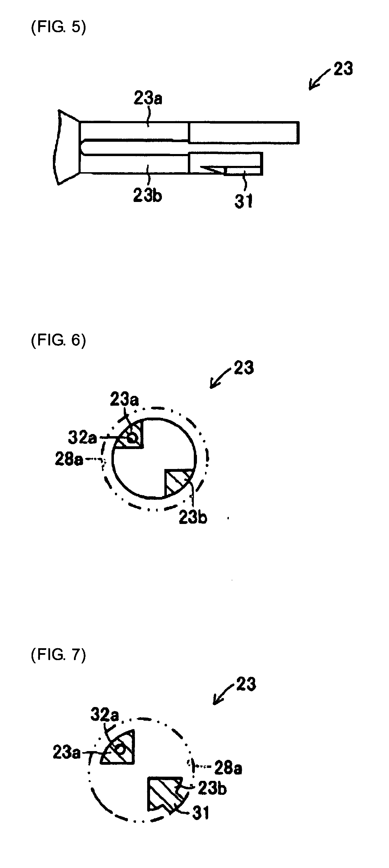

Here of all of those flow paths 13a, etc., that are provided in the medical treatment tube 10, only the portions corresponding to flow paths 13b and 13d are formed. The rod shaped molded portion 23a corresponds to the flow path 13d, and the rod shaped molded portion 23b corresponds to the flow path 13b. Furthermore, the cross-sectional shape of the front end side portion of the land portion 23, as shown in FIG. 7, is formed in the shape in which the protrusion 31 is provided in the center of the circular arc shaped portion in the outer peripheral surface of the rod shaped portion 23b. The protrusion 31 is structured from an inclined portion that is higher (in the direction of separation from the peripheral surface of the rod shaped molded portion 23b) towards the front, with the rear end portion getting gradually wider on the peripheral surface of the rod shaped molded portion 23b. The front edge end of this peripheral side of the protrusion 31 is in contact with the inner peripheral surface of the hollow portion 28a, or forms a slight gap, such that the molding material cannot enter in thereto, with the inner peripheral surface of the hollow portion 28a, where a slit 14b is formed at the portion wherein the protrusion 31 is positioned.

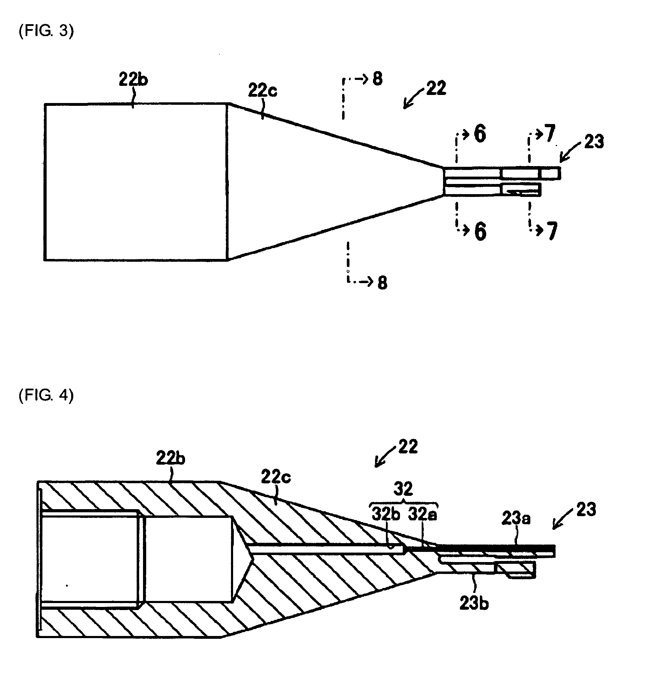

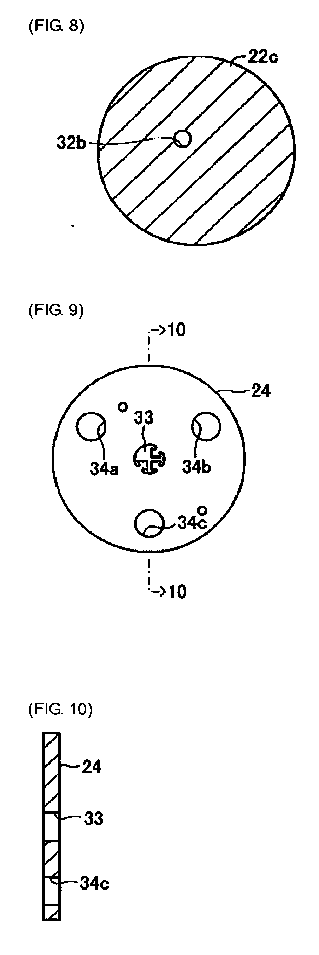

Moreover, no protrusion is provided on the outer peripheral surface of the rod shaped molded portion 23a, where the length, in the axial direction, of the rod shaped molded portion 23a, as shown in FIG. 5, is longer than that of the rod shaped molded portion 23b. Because of this, as described above, the rod shaped molded portion 23b is disposed on the inside of the hollow portion 28a of the bushing 28, and the front end portion of the rod shaped molded portion 23a protrudes towards the front from the hollow portion 28a of the bushing 28. Additionally, the front end portion of an air blowing hole 32, which extends to the rear portion side of the pin 22, is open in the front end surface of the rod shaped molded portion 23a, as shown in FIG. 4 (showing a lateral section surface in a state that is rotated by 45° around the axis in FIG. 3 so that the rod shaped molded portions 23 a and 23b are positioned with one directly above the other). The front end side portion 32a of this air blowing hole 32 is formed so as to be smaller than the rear portion side portion 32b thereof, and FIG. 8 illustrates the rear portion side portion 32b of the air blowing hole 32 that is formed within the tapered portion 22c.

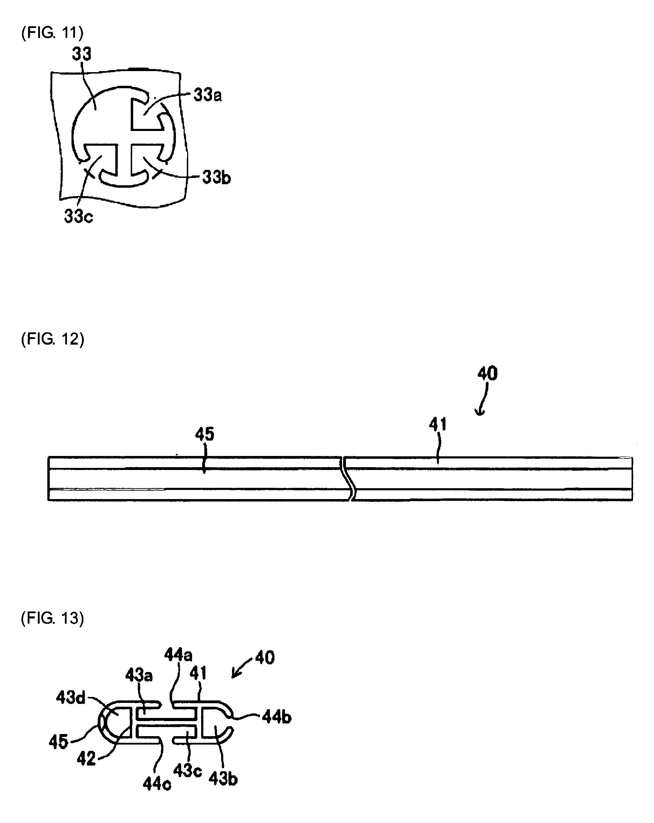

The plate portion 24 is formed in the shape of a disk, as illustrated in FIG. 9 and FIG. 10, where a slit 33, for molding, is formed passing through the center portion thereof in the direction of thickness, where three bolts through holes 34a, 34b, and 34c, which penetrate in the thickness direction, are formed with uniform spacing along the periphery in the outer peripheral side portion. The size of the circular shape (the circular shape that is formed by the peripheral edge portion and the dotted line illustrated in FIG. 11) that is formed when the peripheral edge portion of the molding slit 33 is completed is formed so as to be the same size as the cross section of the hollow portion 28a. Additionally, the shape of the edge portion of the molding slit 33, as illustrated in FIG. 11, is formed in the shape of the edge portion of the end surface of the medical treatment tube 10, with the wedge shaped portion corresponding to the flow path 13d removed.

Additionally, in the portions corresponding to the flow paths 13a, 13b, and 13c in the molding slit 33, closed portions 33a, 33b, and 33c are formed with the same shape as the end surface of the rod shaped molded portion 23b, including the protrusion 31. The plate portion 24 that is formed in this way is secured to the front surface of the bushing 28, using a bolt (not shown), in a state wherein the front end portion of the rod shaped molded portion 23a of the land portion 23 is positioned on the portion corresponding to the flow path 13d of the medical treatment tube 10 in the molding slit 33 (a portion that is maintained at a constant distance from the peripheral edge portion in the upper left portion in the FIG. 11). At this time, the front end surface of the rod shaped molded portion 23b and the protrusion 31 are in contact with the center portion of the rear surface of the plate portion 24, or form a gap therewith so as to be small enough that the molding material does not enter therein.

In this way, a space portion, having the same shape as the end surface of the medical treatment tube 10, is formed by the molding slit 33 and the rod shaped molded portion 23a, in the center portion of the plate portion 24. The front end portion of the front end side molding space portion as set forth in the present invention is formed from this space portion. Note that the edge portion of the molding slit 33 in the plate portion 24 illustrated by the solid line in FIG. 11 forms a portion of the outer shape molding die, where in the peripheral surface at the front end portion of the rod-shaped molded portion 23a that is positioned within the molding slit 33 is a portion for forming the inner shape of the medical treatment tube 10. Additionally, the rear portion side portion of the rod shaped molded portion 23b is a portion that shapes the inner shape of the medical treatment tube 10, where the front end side portion of the rod shaped molded portion 23b is a portion for forming the outer shape of the medical treatment tube 10.

Furthermore, the length of the front end portion wherein the rod shaped molded portion 23a protrudes is set to the same as the thickness of the plate portion 24, and the front end surface of the rod shaped molded portion 23a is positioned so as to be coplanar with the front surface of the plate portion 24. Note that although this is not illustrated, the extrusion molding machine on which the extrusion molding die 20 is mounted is provided with all devices and mechanisms necessary for performing extrusion molding using the extrusion molding die 20, such as a connector between the extrusion molding die 20 and the extrusion molding machine, an adapter for feeding the molding material from the material inlet 29 to the material channel 29 a, a heating cylinder for heating the molding material that is fed into the material inlet 29, a screw that is positioned within the heating cylinder, a control device for controlling each device that is equipped peripherally on the extrusion molding die 20.

When molding the medical treatment tube 10 using the extrusion molding die 20 structured in this way, the extrusion molding die 20 is first heated to the appropriate temperature, following which the molding material that is loaded into a material loading aperture (not shown) is fed by a screw while being heated by the heating cylinder, to pass through an adapter to be filled into the material channel 29a. Following this, the molding material is fed again so that the molding material moves from the material channel 29a into the rear portion side molding space portion. At this time, the molding material is formed into a cylindrical shape in the gap between the inner peripheral surface of the hollow portion 25b and the outer peripheral surface of the straight portion 22b, and then the molding material is transformed into a cylindrical shape with a gradually decreasing diameter while passing through the gap between the inner peripheral surface of the hollow portion 27a and the outer peripheral surface of the tapered portion 22c.

Following this, the molding material moves from the gap between the inner peripheral surface of the hollow portion 27a and the outer peripheral surface of the tapered portion 22c into the rear portion side portion of the front end side molding space portion that is formed from the inner peripheral surface of the hollow portion 28a and the outer peripheral surface of the rear portion side portion of the land portion 23. As a result, the cylindrical molding material fills the entirety of the interior portion of the hollow portion 28a illustrated by the dotted line in FIG. 6, except for the portions of the rod shaped molded portions 23a and 23b. The flow paths 13b and 13d are formed here. Furthermore, in these regions the volume of the molding material per cross-sectional area is greater than that of the front end portion of the gap between the inner peripheral surface of the hollow portion 27a and the outer peripheral surface of the tapered portion 22c.

Additionally, when the molding material moves to the forward side of the rear portion side portion within the front end side molding space portion, the molding material ceases to fill in the portion wherein the protrusion 31 is formed on the inside of the front end side molding space. Because of this, the molding material fills the entirety of the interior portion of the hollow portion 28a illustrated by the dotted line in FIG. 7, except for the portions of the rod shaped molded portions 23a and 23b and of the protrusion 31. The slit 14 is formed here, in addition to the flow paths 13b and 13d. Furthermore, when the forming material reaches the plate portion 24, the forming material is extruded to the outside by passing through the inside of the space portion that is formed from the molding slit 33 and the rod shaped molded portion 23a, while being reduced along the back surface of the plate portion 24.

The molded body that is extruded to the outside is cooled and contracts to approximately 40% to 70% while maintaining essentially a congruent shape, and then is used as the medical treatment tube 10. At the time of the extrusion molding, the molding material is gradually deformed, after heating to the appropriate temperature, while passing from the material channel 29a to the molding slit 33 of the plate portion 24, enabling the forming to be performed easily. When this is done, prior to reaching the plate portion 24, there is a somewhat large amount of molding material accumulated in the rear portion side portion within the front end side molding space portion, and thus an adequate amount of the molding material is supplied to the molding slit 33 of the plate portion 34, so that the medical treatment tube 10 that is produced will be a product with excellent dimensional precision, and, in particular, the wall thicknesses will be as per the design.

As described above, in the medical treatment tube extrusion molding die 20 and extrusion molding method as set forth in the present example of embodiment, the molding material that is loaded into the material channel 29a from the material inlet 29 is first formed into a cylindrical shape in the rear portion side molding space portion, and then gradually approaches the thickness of the medical treatment tube 10. Additionally, the land portion 23 that is positioned within the rear portion side portion of the front end side molding space portion is structured from only a rod shaped molded portion 23a, corresponding to the flow path 13d of the medical treatment tube 10, and a rod shaped molded portion 23b corresponding to the flow path 13b. Because of this, in the rear portion side portion of the front end side molding space there is no portion corresponding to the cross-sectional shape of the medical treatment tube 10, and the molding material also fills into the portions corresponding to the flow paths 13a and 13c. As a result, the situation wherein the volume of molding material that is provided to the molding slit 33 of the plate portion 24 that is positioned at the front end portion of the front end side molding space portion is reduced, preventing the molding of a medical treatment tube 10 with adequate wall thickness, does not occur.

Moreover, the extrusion molding die 20 as set forth in the present example of embodiment enables the formation of a flow path 13d, which is closed on the peripheral surface, through extending the front end side of the rod shaped molded portion 23a to a predetermined portion of the molding slit 33 of the plate portion 24. Furthermore, through establishing the protrusion 31 and the closed portions 33a, 33b, and 33c, the flow paths 13a, 13b, and 13c can be formed connected to the outside through the slits 14a, 14b, and 14c. Because of this, it is possible to form easily the flow paths 13d, having a closed peripheral surface, and flow paths 13a, 13b, and 13c that connect to the outside. Furthermore, given the present example of embodiment, it is possible to mold, with excellent shaping, a medical treatment tube 10 that has a complex shape, even when using polyurethane, which is a soft thermal plastic resin.

### (Second Example of Embodiment)

FIG. 12 and FIG. 13 illustrates a medical treatment tube 40 molded using an extrusion molding die as set forth in a second example of embodiment according to the present invention. The medical treatment tube 40 is structured from an outer peripheral portion 41 that is essentially elliptical in its cross-sectional shape, and a dividing wall portion 42 (the axial portion in the present invention), having an "H"-shaped cross-sectional shape, fabricated along the axial direction of the inner portion of the outer peripheral portion 41. The front end portion of each piece that forms the dividing wall portion 42 is each connected to the inner surface of the outer peripheral portion 41, dividing the inner portion of the outer peripheral portion 41 into four flow paths 43a, 43b, 43c, and 43d. Additionally, slits 44a, 44b, and 44c for connecting the respective flow paths 43a, 43b, and 43c, said slits corresponding to the flow paths 43a, 43b, and 43c in the outer peripheral portion 41, are each formed parallel to the direction in which the dividing wall portion 42 extends.

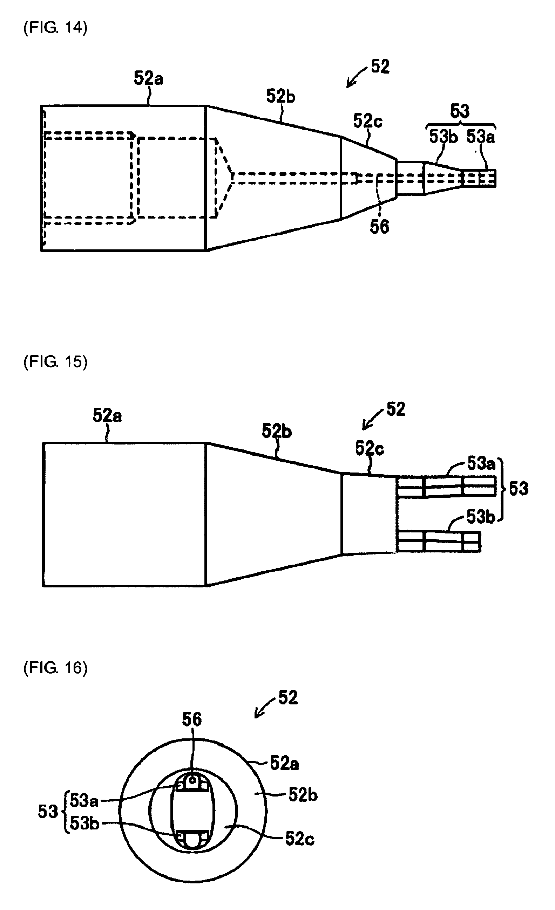

Additionally, a line 45, made from an impermeable material through which x-rays cannot pass, is provided in the portion wherein the flow path 43 is formed on the outer peripheral surface of the medical treatment tube 40. This line 45 is used when confirming, using x-rays, the position of the medical treatment tube 40 that is placed within the body. FIG. 14 through FIG. 16 illustrate the critical portions of a pin 52 (when the securing portion is removed). The pin 52 is structured from: a cylindrical straight portion 52a, which extends forward from the front end of a securing portion (not shown) and is short in the axial direction; a conical tapered portion 52b, which extends forward from the front end of the straight portion 52a and becomes gradually thinner towards the front end side; a flat tapered portion 52c which extends forward from the front end of the tapered portion 52b, gradually becomes more elliptical in its cross-section towards the front end side, and which is flat; and a land portion 53.

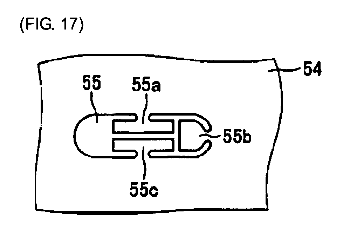

Moreover, although not shown, the outer shape molding die to which this pin 52 is attached not only has the aforementioned hollow portions 27a and 28a of the bushings 27 and 28 formed so as to be compatible with the flat tapered portion 52c and the land portion 53, but also is structured with a flow path provided in a position forward of the bushing holder portion 26 and the bushings 27 in order to provide an impermeable material to the forming space. Additionally, the front end portion of this outer shape molding die is structured from a plate portion 54 that is provided with a molding slit 55 as illustrated in FIG. 17. Furthermore, a gap through which the molding material passes is formed between the outer peripheral surfaces of the straight portion 52a, the tapered portion 52b, and the flat tapered portion 52c, and the inner peripheral surface of the hollow portions of the outer shape molding die corresponding thereto, where the molding space portion on the rear portion side, as set forth in the present invention, is formed by this gap.

Furthermore, the land portion 53 that structures the front end portion of the pin 52 is disposed at the front end portion of the flat tapered portion 52c, and is formed in two rod-shape units as illustrated in FIG. 14 through FIG. 16. That is, land portion 53 is structured from two rod shaped molded portions 53a and 53b that are essentially semicircular in the cross-sectional shape thereof. These two rod shaped molded portions 53a and 53b are formed opposite each other with a gap therebetween, each facing the outside, facing circular arc shaped portions of the two sides in the lengthwise direction of the front end surface of the flat tapered portion 52c, and are disposed in parallel with the axial direction. The rod shaped molded portions 53a and 53b both extend with the same thickness for both the rear portion side portion and the front portion side portion in the front and back directions, but the center portion is structured from a tapered portion that gradually becomes thinner towards the front than at the back, causing the rod shaped molded portions 53a and 53b to be tapered. Furthermore, the front end portion of the rod shaped molded portion 53a is longer than the front and portion of the rod shaped molded portion 53b.

A large gap is formed between outer peripheral surface of the land portion 53 and the inner peripheral surface of the hollow portion of the outer shape molding die, and between the rod shaped molded portions 53a and 53b, where the rear portion side portion of the front end side molding space portion as set forth in the present invention is formed from this gap. That is, the portions that form the flow paths 43a and 43c, of all of those flow paths 43a, etc., that are provided in the medical treatment tube 40, are not provided on the rear end side portion of the front end side molding space portion. Consequently, in the rear portion side portion of the front end side molding space portion, of all of those flow paths 43a, etc., that are provided in the medical treatment tube 40, only the portions corresponding to flow paths 43b and 43d are formed. The rod shaped molded portion 53a corresponds to the flow path 43d, and the rod shaped molded portion 53b corresponds to the flow path 43b. Moreover, the front end portion of an air blowing hole 56, which extends from the rear portion side of the pin 52, is formed on the front end surface of the rod shaped molded portion 53a.

The plate portion 54 is structured provided with a molding slit 55 instead of the molding slit 33 in the plate portion 24. The shape of the edge portion of the molding slit 55 is formed in the shape of the edge of the end of the medical treatment tube 40, with the semicircular portion corresponding to the flow path 43d removed. Additionally, the portions wherein the flow paths 43a, 43b, and 43c, and the slits 44a, 44b, and 44c, are formed in the plate portion 54 are structured from portions 55a, 55b, and 55c that have the same shape as the cross-sectional shape of the respective flow paths 43a, 43b, and 43c, and slits 44a, 44b, and 44c.

The plate portion 24 that is formed in this way is secured to the front surface of the bushing, by a bolt, in a state wherein the front end portion of the rod shaped molded portion 53a is positioned at a portion corresponding to the flow path 43d in the molding slit 55. In this way, a space portion, having the same shape as the end surface of the medical treatment tube 40, is formed by the molding slit 55 and the rod shaped molded portion 53a, in the center portion of the plate portion 54. The front end portion of the front end side molding space portion as set forth in the present invention is formed from this space portion. The other structures in the second example of embodiment aside from the above are identical to those in the first example of embodiment, discussed above.

Moreover, the present example of embodiment enables the molding, through a single injection molding step, of a flat medical treatment tube 40 provided with slits 44a, 44b, and 44 c. Because of this, there is no need for the provision of slits using a machining process after the medical treatment tube 40 is molded. The result is that it is possible to reduce the number of manufacturing processes and to reduce the manufacturing cost. The other effects of the second example of embodiment aside from the above are identical to those in the first example of embodiment, discussed above.

Additionally, the extrusion molding die and extrusion molding method for a medical treatment tube as set forth in the present invention are not limited to the examples of embodiment described above, but rather can be modified as appropriate within the technical scope of the present invention. For example, while in each of the examples of embodiment, described above, the medical treatment tube 10, or the like, was structured having three slits 14a, etc., slits may instead be provided in portions corresponding to all of the flow paths. Additionally, the slits formed in the medical treatment tube 10, or the like, may be one or two slits, or, conversely, no slits may be provided at all.

Furthermore, the flow paths formed in the medical treatment tube are not limited to four, but instead may be another plurality. In addition, the shape of the medical treatment tube is not limited to those of the examples of embodiment described above, but rather may be varied as appropriate. Additionally, it is also possible to provide a line made of an impermeable material, through which x-ray does not pass, in the medical treatment tube 10 as set forth in the first example of embodiment described above. Furthermore, the soft thermoplastic resin from which the medical treatment tube is formed is not limited to polyurethane, but polyvinyl chloride, or other materials may be used instead.

### (Brief Description of the Drawings)

FIG. 1 is an oblique view of a medical treatment tube formed through a first example of embodiment as set forth in the present invention.
FIG. 2 is a cross-sectional diagram of an extrusion molding die.
Fig. 3 is a side view illustrating the tip side portion of the pin.
Fig. 4 is a cross-sectional diagram illustrating the tip side portion of the pin.
FIG. 5 is a side view wherein the land portion is enlarged.
FIG. 6 is a cross-sectional diagram along 6-6 in FIG. 3.
FIG. 7 is a cross-sectional diagram along 7-7 in FIG. 3.
FIG. 8 is a cross-sectional diagram along 8-8 in FIG. 3.
Fig. 9 is a front view of the plate portion.
FIG. 10 is a cross-sectional diagram along 10-10 in FIG. 9.
Fig. 11 is a front view wherein the fabrication slit in the plate portion is enlarged.
FIG. 12 is an side view of a medical treatment tube formed through a second example of embodiment as set forth in the present invention.
FIG. 13 is a front view of an end surface of the medical treatment tube formed through the second example of embodiment.
Fig. 14 is top view of the tip side portion of the pin.
Fig. 15 is a side view illustrating the tip side portion of the pin.
Fig. 16 is a front view of the tip side portion of the pin.
Fig. 17 is a front view wherein the fabrication slit in the plate portion is enlarged.

### (Explanation of Codes)

- 10, 40:: Medical Treatment Tubes
- 12, 42:: Dividing Wall Portions
- 13a, 13b, 13c, 13d, 43a, 43b, 43c, 43d:: Flow Paths
- 14a, 14b, 14c, 44a, 44b, 44c:: Slits
- 20:: Extrusion Molding Die
- 21: Die: Main Unit
- 22, 52:: Pins
- 22c, 52b:: Tapered Portions
- 23, 53:: Land Portions
- 23a, 23b, 53a, 53b:: Rod-Shaped and Molded Portions
- 24, 54:: Plate Portions
- 27a, 28a:: Hollow Portions
- 33, 55:: Molding Slits
- 52c:: Flat Tapered Portion

## Claims

1. An extrusion molding guide for molding a medical treatment tube wherein a plurality of flow paths are formed around an axial portion, comprising:
an outer shape molding die wherein a hollow portion is formed passing through from the rear portion side to the front end side; and
an inner shape molding pin, disposed within the hollow portion of the outer shape molding die, is structured with a land portion so as to form a front end side molding space that is provided with a cross-sectional shape for forming a rear portion side molding space portion between the inner shape molding pin and the inner peripheral wall of the side portion of the rear portion of the hollow portion to form the molding material into a cylinder that is pointed at the front end, wherein the front end portion forms the medical treatment tube between the inner shape molding pin and the inner peripheral surface of the front end side portion of the hollow portion; wherein:
in the cross-sectional shape of the front end side molding space portion, the cross-sectional shape of the front end portion is identical to the cross-sectional shape of the medical treatment tube, and, with the exception of at least one portion of each portion that corresponds to the plurality of flow passages in the land portion, the cross-sectional shape of the rear portion side portion is empty space wherein the molding material can pass therethrough.

2. An extrusion molding die for molding a medical treatment tube as set forth in claim 1, wherein the front end portion of the outer shape molding die is structured from a plate portion provided with an external surface forming hollow portion for forming a surface along the outside of the molding material that moves from the rear portion side molding space portion, wherein a portion of the land extends to a predetermined position within the external surface forming hollow portion of the plate portion so as to form a flow path, the peripheral surfaces thereof being enclosed by the medical treatment tube that is formed, at the position of the land portion that is positioned within the plate portion.

3. An extrusion molding die for molding a medical treatment tube as set forth in claim 1 or claim 2, wherein the cross-sectional shape of the front end portion of the front end side molding space portion can form four flow paths, each connected to the respective front end portion of the space portions of a cross shape in the inner surface of the space portion of a ring shape, formed so that predetermined portions of the space portion of the ring shape may be blocked so that at least one of the four flow paths that can be formed connects to the outside.

4. An extrusion molding die for molding a medical treatment tube as set forth in claim 1 or claim 2, wherein the cross-sectional shape of the front end portion of the front end side molding space portion can form four flow paths, each connected to the respective front end portion of the space portions of an "H" shape in the inner surface of the space portion of a ring shape, formed so that predetermined portions of the space portion of the ring shape may be blocked so that at least one of the four flow paths that can be formed connects to the outside.

5. An extrusion molding die for molding a medical treatment tube as set forth in any one of claim 1 through claim 4, wherein the molding material is a soft thermoplastic resin.

6. A method of extrusion molding a medical treatment tube for molding the medical treatment tube using an extrusion molding die as set forth in any one of claim 1 through claim 5, comprising:
a pointed cylinder molding process for forming the molding material into a cylinder that is pointed at the front end through disposing the inner shape molding pin in the hollow portion of the outer shape molding die and passing the molding material through a rear portion side molding space portion that is a cylinder that is pointed at the front end;
an intermediate shape molding process for molding, at the rear portion side portion of the front end side molding space portion, the cylindrical cross-sectional shape of the molding material, which has been formed in the pointed cylinder molding process, into an intermediate shape between the cross-sectional shape of the medical treatment tube and the cylindrical cross-sectional shape, which is a shape wherein the molding material is filled into a portion wherein at least one of the plurality of flow paths is formed; and
a final shape molding process for forming, at the front end portion of the front end side forming space portion, the cross-sectional shape of the molding material that has been formed in the intermediate shape molding process into the cross-sectional shape of the medical treatment tube.

7. A method of extrusion molding a medical treatment tube as set forth in claim 6, wherein the cross-sectional shape of the molding material that is formed in the final shape forming process is formed into a shape provided with four flow paths, each connected to the respective front end portion of the axial portions that are formed in a cross shape on the inner surface of a ring-shaped portion, wherein a predetermined portion of the ring-shaped portion is cutaway so that at least one of the four flow paths is connected to the outside.

8. A method of extrusion molding a medical treatment tube as set forth in claim 6, wherein the cross-sectional shape of the molding material that is formed in the final shape forming process is formed into a shape provided with four flow paths, each connected to the respective front end portion of the axial portions that are formed in a "H" shape on the inner surface of a ring-shaped portion, wherein a predetermined portion of the ring-shaped portion is cutaway so that at least one of the four flow paths is connected to the outside.

9. A method for extrusion molding a medical treatment tube as set forth in any one of claim 6 through claim 8, wherein the molding material is a soft thermoplastic resin.
